# EUROPEAN PATENT APPLICATION

(11) **EP 3 808 833 A1**
(43) Date of publication of application: **21.04.2021**
(21) Application number: 19818990.4
(22) Date of filing: 13.06.2019
(51) Int. Cl.: C12M 1/00, C12N 1/00

(54) **CELL CULTURING SYSTEM AND CELL CULTURING METHOD**

(30) Priority: 13.06.2018 JP 2018112890
(71) Applicant: IHI Corporation, Koto-ku Tokyo 135-8710 (JP)
(72) Inventor: IKEDA Ryosuke, Tokyo 135-8710 (JP); ISO Yoshiyuki, Tokyo 135-8710 (JP); KAMEKURA Koichi, Tokyo 135-0061 (JP); MIZUNUMA Takato, Tokyo 135-0061 (JP)
(74) Representative: Grünecker Patent- und Rechtsanwälte PartG mbB
(86) International application number: PCT/JP2019/023482
(87) International publication number: WO 2019/240222

(57) **Abstract**

The cell culturing system comprises: a culturing tank housing a liquid culture medium containing cells to be cultured; a fluid force separation device for separating liquid culture medium supplied from the culturing tank into a cell-rich fraction having a relatively high cell density and a cell-poor fraction having a relatively low cell density; and a filtration-separation device for removing cells from the cell-poor fraction separated by the fluid force separation device and recovering the liquid culture medium. The liquid force separator device uses a vortex flow generated by flow along curved flow path having a rectangular cross section to separate the liquid culture medium. Alternatively, the cell-poor fraction from the fluid force separator device is supplied to the culturing tank to reduce the cell density of the liquid culture medium in the culturing tank, and the liquid culture medium is supplied to the filtration-separation device to remove the cells and recover the liquid culture medium.

## Description

### Technical Field

The present disclosure relates to a cell culture system and a cell culture method for efficiently obtaining useful substances through cell culture.

### Background Art

Recently, in a wide range of fields including the pharmaceutical industry, attention has been paid to the use of useful substances such as antibody substances and functional substances produced by animal cells. In order to realize the market supply of useful substances, various ingenuity and improvement have been made in the optimization and efficiency of conditions in cell culture, the isolation and purification method of the produced useful substances, and the like.

Cell culture methods are generally classified into two types: batch type and continuous type. In the batch culture method, a predetermined amount of liquid medium and cells are put into a culture tank, and when the cells grow to a certain concentration, the growth is stopped due to lack of nutrients or poisoning by metabolites. Therefore, the culture is terminated at that point. In the continuous culture method, a predetermined amount of liquid medium and cells are put into a culture tank to proliferate the cells, and at the same time, a part of the liquid medium is extracted and a new liquid medium is put into the replacement. Thus, the cell culture is continued using the supplemented nutrients. Since the extracted liquid medium contains useful substances produced by cells, the useful substances are recovered by appropriately performing purification treatment after removing the cells from the liquid medium.

As a solid-liquid separation means for removing cells from a liquid medium, filtration separation using a separation membrane or centrifugation can be used. Centrifugation requires time for separation, and its practical applicability is by no means high even if a small amount of experimental level separation is good. For this reason, it is common to remove cultured cells from the liquid medium using filtration separation.

Japanese Patent Application Laid-open No. 2017-502666 (Patent Literature 1) relates to an apparatus for cell culture, and describes an acoustic standing wave cell separator which communicates to the outlet of the bioreactor. In the separation by an acoustic standing wave, when applying a high frequency wave from different directions to generate a standing wave, particles are concentrated in the node of the standing wave. Utilizing this phenomenon, cells are separated from the liquid medium by concentrating the cells in the nodes of the standing wave.

On the other hand, as a document relating to the separation of particles contained in a fluid, there is Japanese Patent Application Laid-open 2016-526479 (Patent Literature 2), which describes a hydrodynamic separation device having a curved channel. In this device, a fluid containing particles is supplied to the curved channel, so that the force acting on the fluid flowing the curved channel can be used to separate the particles.

### Citation List

### Patent Literature

Patent Literature 1: Japanese Patent Application Laid-open No. 2017-502666
Patent Literature 2: Japanese Patent Application Laid-open No. 2016-526479

### Summary of Invention

### Technical Problem

The economic rationality of cell culture changes depending on the work efficiency when separating cells from the extracted liquid medium, and the feasibility of practical application depends on it. However, the filtration separation of cells tends to cause clogging of the separation membrane and easily damages the cells. Clogging of the separation membrane can be avoided if the density of cultured cells contained in the liquid medium is low. Therefore, a method is adopted in which the cell density of the liquid medium in the culture tank is maintained at a density at which clogging of the separation membrane can be avoided. However, with this method, it becomes difficult to improve the productivity by cell culture at high density, which is an advantage of continuous culture. Therefore, in order to ensure profitability, it is necessary to control the culture cell density of the liquid medium with extremely high accuracy, and the control of the culture conditions in the culture tank becomes strict.

In addition, during continuous culture, not only the number of living cells increases due to proliferation, but also the number of dead cells increases. In general, when the density of cultured cells in the culture tank exceeds an appropriate range, a part of the liquid medium in the culture tank is discharged as it is, and the cell density is adjusted by adding a new liquid medium. This liquid medium discharged is called "bleed", and useful substances contained in the bleed can be recovered and purified after removing the cells. At the test scale, it is possible to remove cells from bleed through a depth filter, but in practical use, the size and cost of the separation membrane that can handle the bleed with a high density of cells becomes a problem, and thus the reality is that bleeds are discarded.

Regarding the above points, in the apparatus described in Patent Literature 1, the physical influence on the cell due to the action of high frequency waves is large. When cell breakage produces fine cell debris, separation becomes more difficult, which possibly affects the work of purifying useful substances from the recovered liquid medium or reduce purification efficiency.

Since the separation technique described in Patent Literature 2 relates to the separation of solid particles, it is necessary to examine the effect on cells when applied to cell culture.

As described above, the technique for separating and removing cultured cells from the liquid medium is important for putting into practical use the production of useful substances by cell culture. If efficient separation and removal of cells from a liquid medium containing the cells is possible, productivity and workability in continuous culture can be improved and the supply and utilization of substances produced by cells can be expanded.

An object of the present disclosure is to provide a cell culture system and a cell culture method capable of contributing to the practical application of continuous cell culture through a technique for separating cells from a liquid medium and efficiently obtaining useful substances produced by the cells.

### Technical Solution

In order to solve the above problem, a method that can solve the problem of workability due to clogging of the separation membrane was investigated, and it has been found possible to improve the efficiency and workability in continuous culture by using the hydrodynamic separation technique.

According to an aspect of the present disclosure, a subject of the cell culture system is to comprise: a culture tank that contains a liquid medium containing cells to be cultured; a hydrodynamic separation device having a curved flow channel having a rectangular cross-section, and separating the liquid medium supplied from the culture tank into a cell-rich fraction having a relatively high cell density and a cell-poor fraction having a relatively low cell density, using a vortex flow generated by flow through the curved flow channel; and a filtration separator having a filter to remove cells from the cell-poor fraction separated by the hydrodynamic separation device and recover the liquid medium.

The cell culture system may be configured to further comprise: a first return path connecting the hydrodynamic separation device and the culture tank to supply the cell-rich fraction to the culture tank; and a second return path connecting the hydrodynamic separation device and the culture tank to supply the cells removed from the cell-poor fraction to the culture tank. The cell culture system further comprises: a discharge path to discharge a part of the liquid medium containing the cells to the outside as a bleed, and may be configured so that the discharge path is provided as a branch from the first return path, to discard a part or the entire of the cell-rich fraction.

The filter of the filtration separator may be a depth filter, and such a configuration is possible to further comprise: a discharge path to discard the cell-rich fraction to the outside as a bleed. Then, such a configuration is possible to further comprise: a medium replenishment path for supplying a new liquid medium to the culture tank; and an additional filtration separator having a filter for removing the cells from the liquid medium supplied from the culture tank and collecting the liquid medium.

Moreover, according to another aspect of the present disclosure, a subject of the cell culture system is to comprise: a culture tank that contains a liquid medium containing cells to be cultured; a hydrodynamic separation device having a curved flow channel having a rectangular cross-section, and separating the liquid medium supplied from the culture tank into a cell-rich fraction having a relatively high cell density and a cell-poor fraction having a relatively low cell density, using a vortex flow generated by flow through the curved flow channel; a medium return path that connects the hydrodynamic separation device and the culture tank and supplies the cell-poor fraction to the culture tank to reduce the cell density of the liquid medium in the culture tank; and a filtration separator having a filter to remove the cells from the liquid medium supplied from the culture tank and recover the liquid medium.

The cell culture system may be configured to further comprise: a return path that connects the filtration separator and the culture tank and supplies a residual liquid medium containing the cells removed by the filtration separator, to the culture tank. The hydrodynamic separation device has a single inlet for taking in the liquid medium and at least two outlets for discharging the liquid medium separated, wherein the cell-rich fraction is discharged from one of the outlets, and the cell-poor fraction is discharged from the other outlet. The cell culture system may be configured to further comprise: an urging device that urges the liquid medium with a flow pressure for supplying the liquid medium to the hydrodynamic separation device; and a pressure control mechanism that controls pressure environment so that the pressure difference between the liquid medium introduced to the hydrodynamic separation device and the liquid medium derived from the hydrodynamic separation device is equal to or less than a predetermined value.

Moreover, according to an aspect of the present disclosure, a subject of a cell culture method is to comprise: a cell culture for culturing cells in a liquid medium; a hydrodynamic separation to separate the liquid medium supplied from the cell culture into a cell-rich fraction having a relatively high cell density and a cell-poor fraction having a relatively low cell density, using a vortex flow generated by flow through a curved flow channel having a rectangular cross-section; and a filtration separation in which the cells are removed from the cell-poor fraction separated by the hydrodynamic separation with a filter, and the liquid medium is recovered.

Moreover, according to another aspect of the present disclosure, a subject of the cell culture method is to comprise: a cell culture for culturing cells in a liquid medium; a hydrodynamic separation to separate the liquid medium supplied from the cell culture into a cell-rich fraction having a relatively high cell density and a cell-poor fraction having a relatively low cell density, using a vortex flow generated by flow through a curved flow channel having a rectangular cross-section; a medium return in which the cell-poor faction is supplied to the cell culture to reduce the cell density of the liquid medium in the cell culture; and a filtration separator in which cells are removed from the liquid medium supplied from the cell culture with a filter and the liquid medium is recovered.

### Advantageous Effects of Invention

According to the present disclosure, a cell culture system and a cell culture method capable of efficiently separating and removing cells from a liquid medium containing cultured cell to recover the liquid medium and efficiently performing cell culture by taking advantage of continuous culture are provided. Then it is possible to obtain efficiently the useful substances produced by cells.

### Brief Description of Drawings

[FIG. 1] FIG. 1 is a schematic configuration diagram showing the first embodiment of a cell culture system.
[FIG. 2] FIG. 2 is a schematic configuration diagram showing the second embodiment of the cell culture system.
[FIG. 3] FIG. 3 is a schematic configuration diagram showing the third embodiment of the cell culture system.
[FIG. 4] FIG. 4 is a schematic configuration diagram showing the fourth embodiment of the cell culture system.
[FIG. 5] FIG. 5 is a graph showing the relationship between the Dean number and the separation efficiency in cell separation by a hydrodynamic separation device.
[FIG. 6] FIG. 6 is a graph showing the relationship between pumps used in cell separation and cell viability.

### Description of Embodiments

Description for embodiments of the present disclosure will follow, with reference to the accompanying drawings. Note that dimensions, materials, concrete numerical values and the like indicated in the embodiments are only examples for facilitating understanding the contents of the present disclosure and do not limit the present disclosure unless otherwise noted. Moreover, in the description and the drawings of the present disclosure, elements having substantially an identical function and configuration are shown with denoted by identical reference numerals, and overlapped description will be omitted. Elements not directly related to the present disclosure are not illustrated.

One of the separation techniques for separating particles contained in a liquid is one that utilizes the action of a Dean vortex generated in a fluid (see Patent Literature 2 above. Hereinafter, this technique is referred to as hydrodynamic separation). This separation technique utilizes the fact that Dean vortices occur in a liquid flowing through a curved flow channel being curved to one side and having a rectangular cross-section perpendicular to the flow direction, causing a bias in the distribution of particles in the liquid. The particles flowing through the curved flow channel have different distributions in the flow channel depending on their size (see Patent Literature 2). Specifically, a ring-shaped particle distribution is formed in the cross-section of the flow channel, and the particles flow in the flow channel in a spiral shape. At this time, relatively large particles are located on the outside of the ring, and the relatively small particles are located on the inside. Moreover, the distribution form of the particles further changes by setting predetermined separation conditions, and the particles exceeding a certain size converge to the outer circumferential side of the flow channel. Therefore, when dividing the fluid into a fraction on the circumferential side and a fraction on the inner circumferential side, the size and density of the particles contained in the two fractions differ. That is, the fraction on the outer circumferential side contains relatively large particles, and the density of the particles is relatively high. The fraction on the inner circumferential side contains relatively small particles, and the density of the particles is relatively low.

The size of the solid particles converging on the outer circumferential side of the curved flow channel can be adjusted by setting the velocity (flow rate) of the fluid supply and the dimensions of the curved flow channel. Therefore, it is possible to use the hydrodynamic separation for fractionating by the size of the solid particles, or for separating or concentrating the solid particles. Therefore, if the hydrodynamic separation is applied to liquid medium containing cultured cells, it is possible to perform the fractionation according to cell size, or concentration of cells. Then which one to carry out can be determined by setting the conditions for flowing the liquid medium. By setting the separation conditions, it is possible to concentrate most of the cells contained in the liquid medium to the outer circumferential fraction.

In the present disclosure, the liquid medium is supplied to the curved flow channel at a relatively high flow rate when applying the hydrodynamic separation, and the cultured cells contained in the liquid medium are concentrated and separated by utilizing the phenomenon that the cells contained in the liquid medium tend to be ubiquitous on the outer circumferential side of the curved flow channel. While the supplied liquid medium flows through the curved flow channel, relatively large cells are ubiquitous on the outer circumferential side of the curved flow channel. Therefore, a cell-rich fraction having a relatively high cell density (number of cells per volume) generates on the outer circumferential side of the flow channel, and a cell-poor fraction having a relatively low cell density generates on the inner circumferential side. That is, the liquid medium separates into a cell-rich fraction and a cell-poor fraction, and the cell density decreases in the cell-poor fraction. Therefore, when dividing and separating into the cell-rich fraction and the cell-poor fraction at the exit of the curved flow channel, the cell-poor fraction has a low cell density. Therefore, it is possible to perform its filtration separation while avoiding clogging, and the liquid medium can be recovered efficiently from the cell-poor fraction. Since the invasion of cells in hydrodynamic separation is small, the cells contained in the cell-rich fraction can be returned to the culture tank to continue culturing. Regarding the filtration separation of the cell-poor fraction, cell invasion can be reduced by performing filtration separation at a low density, and the cells contained in the residual after separation can be cultured in the culture tank.

As described above, with use of the hydrodynamic separation, the clogging is eliminated in the filtration separation for removing the cultured cells from the liquid medium, and the useful substances contained in the liquid medium can be efficiently recovered. It is also possible to concentrate and dispose of bleed by utilizing the hydrodynamic separation. That is, when the cell density in the liquid medium of the culture tank exceeds a predetermined amount, the liquid medium to be discharged as bleed is extracted and subjected to the hydrodynamic separation. The cells are then concentrated in the cell-rich fraction. Therefore, the amount of liquid medium that is discarded with the cells can be reduced. By returning the cell-poor fraction to the culture tank, the cell density in the culture tank can be reduced to the predetermined amount. Alternatively, the cell-poor fraction can be obtained at a cell density to which filtration separation can be applied, facilitating the application of depth filters and allowing the collection of useful substances by filtering the liquid medium.

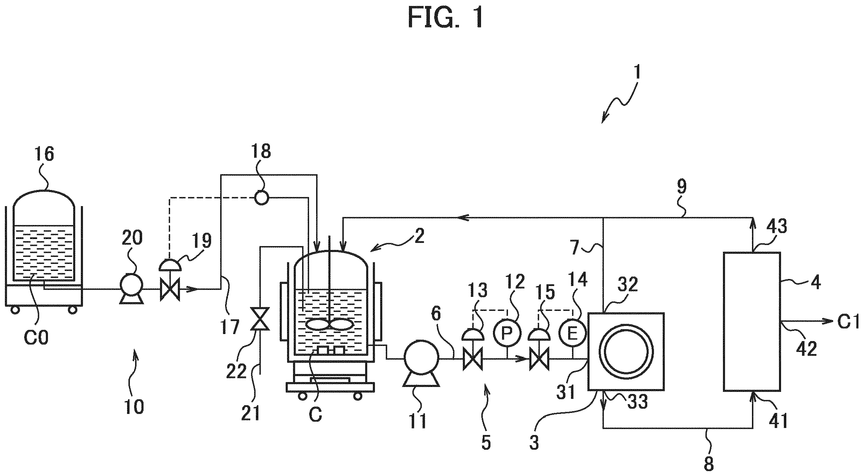

The configuration of the cell culture system will be described below with reference to the first embodiment shown in FIG. 1. The cell culture system 1 of FIG. 1 has a culture tank 2 that contains a liquid medium containing cells to be cultured, a hydrodynamic separation device 3, and a filtration separator 4. The liquid medium C in the culture tank 2 is supplied to the hydrodynamic separation device 3 through a liquid feeding unit 5.

The hydrodynamic separation device 3 has, therein, a curved flow channel having a constant rectangular cross-section perpendicular to the flow direction. It has a single inlet 31 for taking in the liquid medium C at one end of the curved flow channel, and at least two outlets 32 and 33 for separating and discharging the liquid medium at the other end of the curved flow channel. The hydrodynamic separation device 3 utilizes a vortex flow generated in the liquid swirling in one direction by flowing through the curved flow channel, to move the cells contained in the liquid medium C and make them ubiquitous on the outside (outer circumferential side) of the flow channel. Therefore, by dividing the liquid medium discharged from the curved flow channel into a fraction on the outer circumferential side and a fraction on the inner circumferential side, it separates into a cell-rich fraction with a relatively high cell density and a cell-poor fraction with a relatively low cell density. The liquid medium having a relatively high cell density is discharged from one outlet 32, and the liquid medium having a relatively low cell density is discharged from the other outlet 43.

The curving shape of the curved flow channel of the hydrodynamic separation device 3 includes a substantially circumferential shape, a substantially arc (partial circumference) shape, a spiral shape, and the like, and any of these shapes may be used. The hydrodynamic separation device 3 can be designed with a flow channel unit having one curved flow channel as a constituent unit. Specifically, as a flow channel unit, a flat layer-shaped molded body in which one curved flow channel is formed therein is formed of plastic or the like. At that time, it is configured so that both ends of the curved flow channel open to the end face of the molded body to have one inlet and at least two outlets. Using such a molded body as a flow channel unit, a hydrodynamic separation device can be configured by one flow channel unit or a combination of a plurality of flow channel units. By stacking a plurality of flow channel units to form a parallel-shaped flow channel, the processing flow rate of the liquid medium can be increased.

The liquid feeding unit 5 has a supply path 6 composed of a pipe connecting the culture tank 2 and the hydrodynamic separation device 3, and the liquid medium C containing the cells in the culture tank 2 is sent to the hydrodynamic separation device 3 through the supply path 6. The cell culture system 1 of FIG. 1 has a pipe connecting the outlet 32 of the hydrodynamic separation device 3 and the culture tank 2, whereby a first return path 7 for returning the cell-rich fraction to the culture tank is configured. The supply path 6 and the first return path 7 form a circulation system capable of circulating the liquid medium between the culture tank 2 and the hydrodynamic separation device 3.

The cell-rich fraction separated by the hydrodynamic separation device 3 is returned to the culture tank 2 through the first return path 7, and the cell culture is further continued. The cell-poor fraction (fraction on the inner circumferential side) is supplied from the filtration separator 4 through a supply path 8.

The filtration separator 4 has a single inlet 41 for taking in the liquid medium and at least two outlets 42 and 43 for separating and discharging the liquid medium, and contains therein a filter capable of separating cells from the liquid medium. The supply path 8 is connected to the inlet 41, and the cell-poor fraction is supplied from the hydrodynamic separation device 3. The cell-poor faction is separated into a liquid medium C1 that permeates the filter and the remaining liquid medium, and the cells removed by the filter are concentrated in the remaining liquid medium to increase the cell density. The liquid medium C1 that has passed through the filter is discharged from the outlet 42 and the remaining liquid is discharged from the outlet 43. Since the liquid medium contains useful substances produced by cultured cells, the liquid medium C1 discharged from the outlet 42 is subjected to a separation treatment and a purification treatment for recovering the useful substances as necessary.

In the present disclosure, the liquid medium whose cell density has been reduced by the hydrodynamic separation device is supplied to the filtration separator 4. Therefore, a filtration-separation device generally used for separating cultured cells can be preferably used as the filtration separator 4, and the liquid medium can be recovered satisfactorily while avoiding clogging. From the viewpoint of preventing cell invasion, a filtration separation device of cross-flow filtration type is suitable, and examples of the structure of the filter module include a multilayer plate structure, a spiral sheet structure, and a hollow fiber bundle structure. As a filter for separating cells, it can be selected from various filters made of materials having an appropriate pore size and suitable for cell treatment, and the types of the filters include separation membranes such as microfiltration membranes and ultrafiltration membranes. A microfiltration membrane having a pore size of about 0.01 to 10 µm is suitable for removing cells, and when a microfiltration membrane having a pore size of about 0.2 to 0.45 µm is used as the filter, the cells can be separated efficiently. When a filter with hydrophilization treatment on the surface is used, it is easy to suppress cell damage and clogging. If necessary, a plurality of filters having different pore diameters may be used, or may be configured for multi-step separation. At this time, it is also possible to remove metabolites, micro-condensates of waste products, dead cell fragments (debris), etc. generated by cell culture, from the liquid medium. Depending on the object to be removed, for example, a microfiltration membrane, an ultrafiltration membrane, or the like can be selected with an appropriate pore size.

The cell culture system 1 has a pipe connecting the outlet 43 of the filtration separator 4 and the culture tank 2, whereby a second return path 9 for supplying the cell removed from the cell-poor fraction to the culture tank 2 is configured. In this embodiment, the second return path 9 merges with the first return path 7 to form part of the circulation system. However, it may be configured to supply to the culture tank 2 independently without merging with the first return path 7.

Further, in order to replenish the culture tank 2 with a new liquid medium C0 corresponding to the amount of the liquid medium C1 discharged from the filtration separator 4, a medium supplement unit 10 is provided. The liquid medium C in the culture tank 2 is maintained at a constant amount by replenishing the new liquid medium C0.

The cell separation efficiency in the hydrodynamic separation device 3 varies depending on the Dean number and the pressure in the liquid supplied to the curved flow channel, and there are appropriate ranges of the Dean number and the pressure capable of suitable separation. The Dean number De is expressed by the formula: De = Re (D/2Rc)^{1/2} (Re: Reynolds number (-), D: representative length (m), Rc: turning radius of the flow channel (m)). Since the Dean number is proportional to the flow rate of the liquid, suitable cell separation is carried out by appropriately controlling the flow rate and pressure of the liquid supplied to the hydrodynamic separation device. In general, the Dean number is preferably 30 or more and 100 or less, and more preferably about 50 to 80. Therefore, the flow velocity (flow rate) of the liquid medium is set so that the Dean number is in such a range.

The liquid feeding unit 5 of the cell culture system 1 has an urging device for urging the liquid medium C with a flow pressure for supplying the liquid medium C to the hydrodynamic separation device 3, specifically, a pump 10. The flow rate and flow pressure of the liquid medium C supplied to the hydrodynamic separation device 3 change depending on the flow pressure applied by the pump 10. Cell viability is important for returning the isolated cells to the culture tank 2 to continue cell culture. In this regard, the viability of cells varies depending on the magnitude of pressure fluctuation during separation in the hydrodynamic separation device 3. That is, in order to prevent a decrease in the viability, it is effective to reduce pressure fluctuation in the cell separation. Therefore, on the basis of this point, the pressure environment in which the liquid feeding unit 5 flows the liquid medium C through the hydrodynamic separation device 3 is controlled so as to suppress the decrease in cell viability caused by pressure fluctuation in the liquid medium while flowing through the hydrodynamic separation device 3.

Therefore, the liquid feeding unit 5 has a pressure control mechanism, whereby the pressure environment is controlled so that the pressure difference between the liquid medium introduced to the hydrodynamic separation device 3 and the liquid medium derived from the hydrodynamic separation device 3 becomes equal to or less than a predetermined value. The pressure control mechanism can be configured by a pressure gauge and a pressure-regulating valve. Specifically, in the cell culture system 1 of FIG. 1, a pressure gauge 12 and a pressure-regulating valve 13 are provided in the supply path 6.

Animal cells are resistant even under relatively high pressure, and the viability of the cells is maintained even under a pressure supply of, for example, about 1 MPa. However, if the pressure fluctuation is large, even at a supply pressure (inlet pressure) of about 0.6 MPa, the cell damage is large and the viability decreases. Therefore, it is suitable to control the supply of the liquid medium so that the fluctuation of the pressure applied to the cells (difference between the inlet pressure and the outlet pressure) during the separation is equal to or less than a predetermined value. Specifically, the pressure fluctuation (pressure difference) is controlled to be less than 0.6 MPa, and it is preferable to set it to 0.45 MPa or less, more preferably 0.40 MPa or less. Under the conditions of separation in which pressure fluctuations are suppressed as described above, the cell viability can be maintained at about 98 % or more. Therefore, with returning the concentrated and separated cells into the culture tank, proliferation efficiency can be maintained.

In the cell culture system 1, the pressure on the outlet side at the outlets 32 and 33 of the hydrodynamic separation device 3 is released to atmospheric pressure. Therefore, the pressure difference between the introduced liquid medium and the derived liquid medium is equal to the pressure (gauge pressure) measured by the pressure gauge 12. Therefore, pressure control can be performed based on this measured value. The pressure environment is controlled so that the pressure difference is less than 0.60 MPa, in order to prevent the decrease in viability in the cells separated. It is preferably controlled to be 0.45 MPa or less, more preferably 0.40 MPa or less.

If the pressure in the liquid medium discharged from the hydrodynamic separation device 3 is high, the upper limit of the pressure range applicable to the liquid medium introduced into the hydrodynamic separation device 3 becomes high. Therefore, even for the condition setting that the pressure applied to the liquid medium introduced into the hydrodynamic separation device 3 is set to 0.6 MPa or more, it is possible to deal with it by reducing the pressure difference between the introduction and the discharge of the liquid medium. That is, pressure-regulating valves may be provided in the first return path 7 and the supply path 8 to increase the outlet pressure in the liquid medium discharged from the outlets 32 and 33 of the hydrodynamic separation device 3 and reduce the pressure difference. In this case, it is preferable to install pressure gauges in the first return path 7 and the supply path 8 to monitor the discharge pressure, so as to obtain an appropriate pressure difference. At this time, it is suitable to confirm the pressure environment so that the liquid medium flowing through the first return path 7 does not undergo a sudden pressure fluctuation. However, the pressure in the first return path 7 and the supply path 8 is set in consideration of a range suitable for separation process of the filtration separator 4.

As described above, the separation efficiency in the hydrodynamic separation device 3 depends on the flow velocity of the liquid medium flowing through the curved flow channel. Therefore, the liquid feeding unit 5 further has a flow rate-controlling mechanism for controlling a flow rate of the liquid medium supplied to the hydrodynamic separation device 3. The flow rate of the liquid medium flowing through the supply path 6 is controlled so that the liquid medium flowing through the curved flow channel of the hydrodynamic separation device 3 has an appropriate flow rate. The flow rate-controlling mechanism is composed of a flow meter 14 and a flow rate-adjusting valve 15. The flow meter 14 monitors the flow rate of the liquid medium C supplied to the hydrodynamic separation device 3, and the flow rate-adjusting valve 15 adjusts the flow rate of the liquid medium C supplied to the hydrodynamic separation device 3, based on the flow rate monitored by the flow meter 14. The flow rate of the liquid medium supplied to the hydrodynamic separation device 3 is adjusted so that a liquid medium having a cell density suitable for filtration separation is supplied to the filtration separator 3.

In the cell culture system 1, if it is possible to adjust appropriately the flow rate of the liquid medium supplied to the hydrodynamic separation device 3 by means of drive control of the pump 11, the flow rate-adjusting valve 15 may be omitted. Further, when the supply pressure of the liquid medium supplied to the hydrodynamic separation device 3 can be adjusted appropriately by the drive control of the pump 11, the pressure-regulating valve 13 can be omitted. Therefore, the configuration of the cell culture system is possibly simplified by appropriately designing the dimensions of the supply path 6 and the first return path 7 based on the processing capacity (size of the cross-section of the flow channel and the number of flow channels) in the hydrodynamic separation device 3.

The medium supplement unit 10 of the cell culture system 1 has a medium tank 16 for accommodating a new liquid medium C0 and a medium replenishment path 17 that connects the medium tank 16 and the culture tank 2. The culture tank 2 is replenished with a new liquid medium C0 of the medium tank 16 to maintain a constant amount of the liquid medium in the culture tank 2. That is, a new liquid medium C0 corresponding to the amount of the liquid medium C1 recovered from the filtration separator 4 is replenished from the medium tank 16 through the medium replenishment path 17. For this purpose, in the cell culture system 1 of FIG. 1, the medium supplement unit 10 has a liquid level meter 18 installed in the culture tank 2 and a flow rate-adjusting valve 19 installed in the medium replenishment path 17. The flow rate-adjusting valve 19 is controlled according to the liquid level detected by the liquid level meter 18, and the amount of liquid medium C0 supplied by a tubing pump 20 attached to the medium tank 16 is adjusted so that the liquid level of the liquid medium in the culture tank 2 is kept constant. The amount of liquid medium returned from the hydrodynamic separation device 3 and the filtration separator 4 to the culture tank 2 is reduced by the amount of the liquid medium C1 recovered from the filtration separator 4. Therefore, by maintaining the liquid level in the culture tank 2, replenishment corresponding to the recovered liquid medium C1 is performed.

Such replenishment can be performed also by using a weighing scale for measuring the weight of the culture tank 2 instead of the liquid level meter, and a new liquid medium may be replenished so that the weight is kept constant. Alternatively, a measuring device for measuring the weight or flow rate of the liquid medium recovered from the filtration separator 4 may be used. Or, a measuring instrument (flow meter) for measuring the amount of the cell-rich fraction of the first return path 7 returned to the culture tank 2 and the amount of the liquid medium of the second return path 9 returned from the filtration separator 4 may be used to perform such replenishment.

The culture tank 2 and the medium tank 16 are containers capable of preventing microbial contamination. Each of these is equipped with a heater or a cooler and a temperature control function, and the internal liquid medium is maintained at a temperature suitable for cell culture or storage. The culture tank 2 is provided with a stirrer capable of stirring at an appropriate speed that does not damage the cells, and homogenizes the liquid medium. In addition, if necessary, those having a function of adjusting the amounts of oxygen/carbon dioxide/air, pH, conductivity, light amount, etc. can be appropriately used so that the culture environment can be adjusted to be suitable for the cells to be cultured.

A discharge path 21 for discharging the liquid medium containing the proliferated cells to the outside as a bleed is connected to the culture tank 2, and, when the cells proliferate excessively in the liquid medium of the culture tank 2, a part of the liquid medium is discharged from the discharge path 21 to the outside. In response to this discharge amount, a new liquid medium C0 is supplied from the medium tank 16, and the amount of the liquid medium in the culture tank 2 is maintained at a predetermined amount, whereby the cell density of the liquid medium is reduced. An on-off valve 22 is provided in the discharge path 21, and the amount of bleed discharged can be adjusted by this opening and closing. Therefore, the cell density of the liquid medium in the culture tank 2 can be adjusted to an appropriate amount. In the cell culture system 1, the liquid medium to be separated in the filtration separator 4 is a cell-poor fraction in which the cell density is reduced in the hydrodynamic separation device 3, so that the cell density of the liquid medium in the culture tank 2 may be higher than the cell density of the liquid medium suitable for filtration separation. That is, the upper limit of the cell density of the liquid medium in the culture tank 2 can be set high in consideration of the separation efficiency in the hydrodynamic separation device. Therefore, the cell density of the liquid medium discharged as bleed is also increased, and the loss of the liquid medium due to the disposal of bleed is reduced. Since it is possible to improve the viability by periodically extracting the bleed when the viability of the cells decreases, it is very useful to reduce the amount of the liquid medium discharged as the bleed.

As described above, in the cull culture system, the pressure environment of the liquid medium supplied to the hydrodynamic separation device 3 is controlled in order to preferably maintain the viability of the cells. Since the viability of cells is also affected by the pump 11 that supplies the flow pressure to the liquid medium containing the cells, it is preferable to select a suitable liquid feeding means as the pump 10 in order to maintain the cell viability. In order to suppress the influence on the viability of cells, it is preferable to use a pump of a type that does not apply shearing force to the cells. Specifically, it is suitable to use a positive displacement pump that pushes out a constant volume of liquid by utilizing a volume change due to reciprocating motion or rotary motion. Examples of the positive displacement pump include reciprocating pumps such as piston pumps, plunger pumps, diaphragm pumps and wing pumps, and rotary pumps such as gear pumps, vane pumps and screw pumps. As an embodiment, something is given that utilize a pressurizing tank equipped with a compressor. By pressurizing the liquid medium contained in the pressurizing tank with the compressor, the liquid medium can be pumped from the pressurizing tank to the hydrodynamic separation device.

The concentration state of cells and fractionating yield in the hydrodynamic separation device differ depending on the dividing position of the outlets at the exit. That is, the division ratio between the factions of outer circumferential side and the inner circumferential side can be adjusted by designing the dividing position of the outlets at the terminal exit of the curved flow channel. In the cell culture system of FIG. 1, the hydrodynamic separation device has two outlets as outlets of the curved flow channel, but may be divided into three or more outlets. When the terminal exit is divided into three or more outlets, the amount of the fraction to be returned to the culture tank and the amount of the fraction supplied to the filtration separator can be changed depending on the situation.

The cell culture method that can be carried out in the cell culture system 1 as mentioned above is described below. The cell culture method includes a cell culture step of culturing cells in a liquid medium, a hydrodynamic separation step using a curved flow channel, and a filtration separation step using a filter. The cell culture step is carried out in the culture tank 2, and the hydrodynamic separation step is carried out in the hydrodynamic separation device 3. The filtration separation step is carried out in the filtration separator 4. In the hydrodynamic separation step, the liquid medium supplied from the cell culture step is separated into a cell-rich fraction and a cell-poor fraction by utilizing a vortex flow generated by flow through a curved flow channel having a rectangular cross-section. The cell-rich fraction has a relatively high cell density, and the cell-poor fraction has a relatively low cell density. In the filtration separation step, the cells are removed from the cell-poor fraction separated by the hydrodynamic separation step to recover the liquid medium.

In the cell culture step, cell culture is started in a liquid medium adjusted to an environment suitable for the cultured cells, and the glucose concentration and cell density of the liquid medium are monitored during the culture. As the culture progresses, glucose is consumed and the cells proliferate so that the cell density increases. Along with this, useful substances such as antibodies produced by cells increase. When the glucose concentration in the cell culture step starts to fall below a predetermined concentration, for example, 1 g/L, a new liquid medium is continuously supplied. At this time, the supply is adjusted so as to be within a range of a predetermined concentration or less that does not cause glucose depletion and excess. At the same time, the pump 11 is operated to start supplying the liquid medium to the hydrodynamic separation step and the subsequent filtration separation step. Then the liquid medium having the increased cell density is returned from the hydrodynamic separation step and the filtration separation step. When the liquid medium C1 recovered from the filtration separation step contains a useful substance having a desired concentration, it is recovered and the useful substance is separated and purified. During this time, the supply to the hydrodynamic separation step is controlled so that the amount of the liquid medium C1 recovered from the filtration separation step corresponds to the amount of the new liquid medium C0 supplied to the culture step.

When the concentration of the useful substance in the liquid medium recovered from the filtration step is low, it may be discarded or supplied to the culture tank 2 for reuse in consideration of its glucose concentration and the like. For example, if the supply flow rate of the new liquid medium required for the culture tank is low and the amount of liquid medium in the culture tank cannot be kept constant by continuous processing while maintaining the rated flow rate in the hydrodynamic separation step, the supply to the hydrodynamic separation step may be performed intermittently. As a result, the amount of liquid medium in the culture tank can be maintained while maintaining the rated flow rate in the hydrodynamic separation step and performing suitable separation. When intermittent hydrodynamic separation is performed, increasing the frequency of switching the supply to the hydrodynamic separation step and performing fine intermittent operation are effective in suppressing the concentration fluctuation in the liquid medium of the culture tank.

When the liquid medium in the cell culture step reaches a predetermined cell density, an excess amount of cells are discharged as bleeds based on the prediction of an increase in cell density due to proliferation. At the same time, the supply amount of the new liquid medium is adjusted to maintain the liquid amount and cell density of the liquid medium in the culture tank at predetermined amounts.

The cell density may be measured by sampling the liquid medium or directly measured with a detector of a cell measuring apparatus. In the cell culture system 1, the liquid medium supplied to the filtration separation step is a cell-poor fraction obtained by hydrodynamic separation, so that the cell density of the liquid medium in the cell culture step can be set higher than the cell density of the liquid medium suitable for filtration separation. The cell density suitable for filtration separation varies depending on the filter used, etc., but it may be generally about 5 ×10⁷ cells/mL, and a higher cell density can be set as a predetermined amount in the cell culture step, considering the separation in the hydrodynamic separation. Therefore, the amount of liquid medium discharged as bleed can be reduced.

The supply pressure of the liquid medium is controlled by using the pressure gauge 12 and the pressure-regulating valve 13 so that the cell viability does not decrease due to the pressure fluctuation in the liquid medium while flowing through the curved flow channel. At this time, the pressure fluctuation (pressure difference) in the hydrodynamic separation is adjusted to be less than 0.60 MPa, preferably 0.45 MPa or less, and more preferably 0.40 MPa or less.

In the separation process, the liquid medium containing the cells is introduced into the curved flow channel having a rectangular cross-section from a single inlet, and supplied to the curved flow channel in a uniform state. The curved flow channel of the hydrodynamic separation device is a flow channel having a rectangular cross-section (radial cross-section) perpendicular to the flow direction. While the uniform liquid medium flows through the curved flow channel, relatively small cells and fine particles rest on the Dean vortex and change their distribution in a circular motion in the rectangular cross-section. On the other hand, for relatively large cells, the lift that stays on the outer circumferential side of the flow channel acts relatively strongly, so that the distribution is concentrated on the outer circumferential side. When this action becomes stronger, the tendency of ubiquity to the outer circumferential side of the cells increases. At the end of the curved flow channel, a cell-rich fraction having a relatively high cell density, that is, a liquid medium in which the cells are concentrated, is discharged from the outlet 32 on the outer circumferential side. From the outlet 33 on the inner circumferential side, the remaining liquid medium, that is, a cell-poor fraction in which the cells are decreased and the cell density is relatively low is discharged.

As shown in the above-described formula, the Dean number varies depending on the turning radius Rc of the curved flow channel and the cross-sectional dimension of the flow channel (the representative length D in the above formula can be regarded as the width of the curved flow channel). Therefore, the Dean number can be adjusted to a suitable value based on the design of the curved flow channel, whereby the hydrodynamic separation device is capable of carrying out concentrated separation of cells with good separation efficiency. Further, the flow rate of the fluid can be adjusted by setting either the width (radial direction) or the height of the cross-section of the curved flow channel. Thus, the hydrodynamic separation device can be configured, based on the design of the curved flow channel, so that the cell separation process can be performed at an appropriate pressure and a desired flow rate. Therefore, the design of the curved flow channel can be appropriately changed so as to enable suitable separation according to the conditions of the separation target (cell size distribution, medium viscosity, etc.). From the viewpoint of cell separation efficiency, it is suited that the curved flow channel has a rectangular cross-section having an aspect ratio (width/height) of 10 or more. By supplying the liquid medium to such a curved flow channel at a flow rate of approximately 10 to 500 mL/min, the cells are well in ubiquitous, and concentration and separation of the cells can be performed with an efficiency of about 5 to 25 billion cells/min. When such hydrodynamic separation is applied to, for example, a liquid medium having a cell density of about 5 ×10⁷ cells/mL, it can be separated into a cell-rich fraction having a cell density of about 7 ×10⁷ cells/mL or more and a cell-poor faction having a cell density of about 1 ×10⁷ cells/mL or less.

The division ratio of the cell-rich fraction on the outer circumferential side and the cell-poor fraction on the inner circumferential side can be adjusted by the dividing position of the outlets at the terminal exit of the curved flow channel of the hydrodynamic separation device. Based on this, the degree of concentration and separation of cells can be adjusted. In general, when the cross-sectional area ratio at the flow channel exit is designed so that the division ratio (volume ratio) of the outer circumferential-side fraction/inner circumferential-side fraction is about 90/10 to 50/50, it is suitable for concentrated separation of cells as described above. Such separation is possible that concentrates about 95 % or more of cells into a cell-rich fraction. The cell-rich fraction discharged from the hydrodynamic separation step is returned to the cell culture step in the culture tank, and the cell-poor fraction is supplied to the filtration separation step. Larger cells have higher viability and activity than smaller cells, and the cell-rich fraction contains a relatively large number of large cells. Therefore, when the cell-rich fraction is returned to the cell culture step and the liquid medium circulates between the cell culture step and the hydrodynamic separation step, the cell culture efficiency is increased.

In the filtration separation step, a filter having a pore size that does not allow cells to permeate is used, and the liquid medium C1 that has permeated the filter is recovered. Microfiltration membranes generally have a pore size of about 0.01 to 10 µm and are suitable for cell separation and removal. When the pore size is about 0.2 to 0.45 µm, the separation between the cells and substances such as proteins and polysaccharides is good. For cell filtration separation, it is suitable to apply a cross-flow filtration method in which a liquid medium is allowed to flow parallel to the membrane surface. Since the cells removed by the filter are concentrated in the remaining liquid medium from which the liquid medium C1 has been recovered, the cell density in the remaining liquid medium is higher than the cell-poor fraction. The remaining liquid medium is returned to the cell culture step to continue culturing. In the filtration separation, the supply flow rate and supply pressure of the cell-poor fraction are appropriately adjusted in consideration of cell damage due to filtration pressure and shear stress. Since the cell density of the cell-poor fraction is low, the load on filtration is small and clogging is eliminated.

The liquid medium C1 collected through the filter is subjected to a treatment for separating and purifying useful substances, if necessary. As a result, metabolites, micro-condensates of waste products, dead cell fragments (debris) and the like produced by cell culture are removed from the liquid medium. For the purpose of performing a part of such separation and purification, the filtration separation step may be configured for multi-step separation using a plurality of filters having different pore sizes. For example, an ultrafiltration membrane having a pore size of about 0.001 to 0.01 µm allows amino acids, peptides, saccharides, antibiotics and the like to permeate, and can remove macromolecules, proteins, polysaccharides and the like.

The total amount of liquid medium returned from the hydrodynamic separation step and the filtration separation step to the culture step is reduced by the amount of the liquid medium recovered as a filtrate in the filtration separation step. Therefore, the supply amount of a new liquid medium C0 is adjusted so that the liquid medium C0 corresponding to this amount is replenished in the culture step. As a result, the nutrients used by the cells are continuously replenished and the concentration of the metabolite is diluted, which enables continuous culturing of the cells.

The cells return from the hydrodynamic separation step and the filtration separation step to the cell culture step, and the cell culture is continued. The cell density of the liquid medium in the culture tank is monitored. In a state where the cell density of the liquid medium is maintained at a predetermined amount, the amount of new liquid medium C0 supplied from the medium tank corresponds to the amount of liquid medium recovered from the filtration separation step. When the cells proliferate excessively and the cell density exceeds a predetermined about, a part of the liquid medium C is discharged as bleed to adjust the cell density. Therefore, the supply amount of the new liquid medium C0 increases correspondingly. In this way, the amount of the liquid medium in the culture tank 2 is kept constant, and the cell density of the liquid medium is reduced to the predetermined amount. The amount of bleed emission can be determined based on predicted cell growth. Based on the monitoring result of the cell density, the emission amount may be appropriately modified so that the cell density is maintained at the predetermined amount. The cell density of the liquid medium in the culture tank 2 can be set higher than the cell density of the liquid medium suitable for filtration separation, and the predetermined amount of the cell density in the culture tank may be determined in consideration of the degree of separation in the hydrodynamic separation. Therefore, the cell density of the liquid medium discharged as the bleed is also increased, and the loss of the liquid medium due to the disposal of bleed decreases. After the desired culture time has passed, the culture may be stopped as appropriate. Usually, the culture can be continued for about 10 to 30 days.

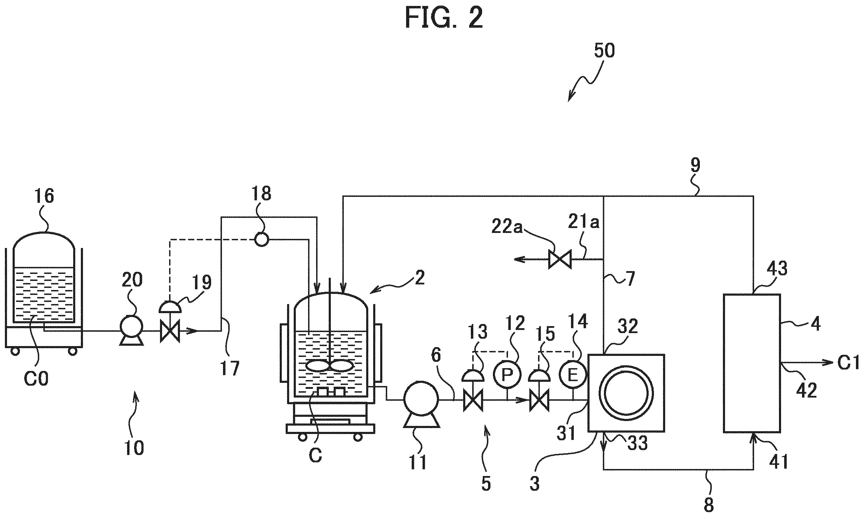

The cell density in the cell-rich fraction obtained by hydrodynamic separation is higher than the cell density in the liquid medium of the culture tank. Therefore, the cell-rich fraction is convenient for disposal as the bleed. As a second embodiment of the cell culture system, FIG. 2 shows a cell culture system configured to discharge the cell-rich fraction as the bleed.

In the cell culture system 50 of FIG. 2, the discharge path 21a for discharging the bleed is provided so as to branch off from the first return path 7 for discharging the cell-rich fraction from the hydrodynamic separation device 3, instead of branching from the culture tank 2. An on-off valve 22a is installed in the discharge path 21a, and the on-off valve 22a is controlled so as to discard the cell-rich fraction when the cell density of the liquid medium in the culture tank 2 exceeds the predetermined amount. Alternatively, a flow rate-adjusting valve may be installed instead of the on-off valve 22a so that the proportion of the cell-rich fraction discharged as the bleed can be adjusted and changed. In this way, a part or the whole of the cell-rich fraction discharged from the hydrodynamic separation device can be discharged from the discharge path 21a as a bleed.

In the hydrodynamic separation device 3, there is an optimum operating flow rate (rated flow rate) that maximizes the separation efficiency. Therefore, when the flow rate to be discharged as the bleed is small, it can be dealt with by controlling the on-off valve 22a so as to intermittently discharge the cell-rich fraction from the discharge path 21a. Alternatively, the operation itself of the hydrodynamic separation device 3 can be performed intermittently. Since the new liquid medium is supplied to the culture tank 2 in response to the discharge of the bleed, the liquid quality of the liquid medium in the culture tank fluctuates. Therefore, in the case of intermittent bleed discharge, it is advisable to increase the switching frequency and make fine adjustments so that the fluctuation of the liquid quality of the liquid medium in the culture tank 2 is as small as possible. Since the cell culture system 50 is the same as the cell culture system 1 of FIG. 1 except for the configuration for discharging the bleed, the description of the same device and members will be omitted.

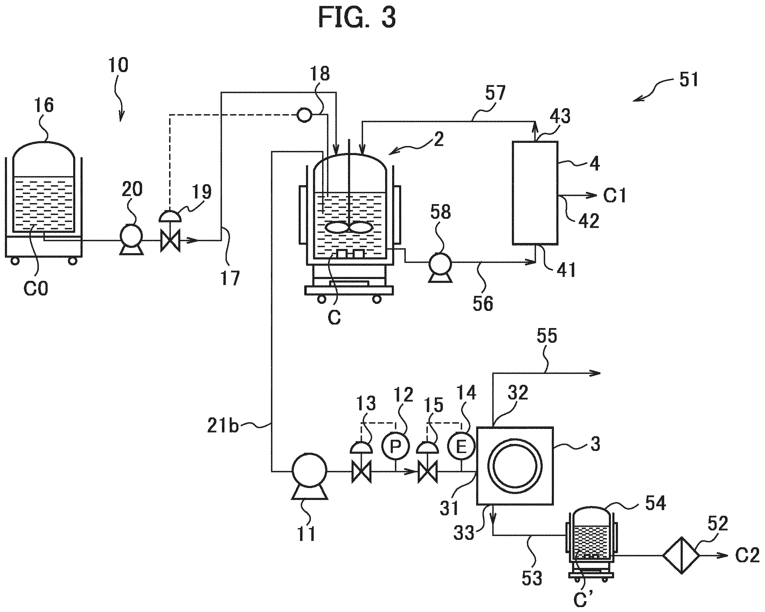

The embodiments of FIG. 1 and FIG. 2 are configured to combine the hydrodynamic separation with filtration separation to recover a liquid medium containing useful substances, in which the load on filtration separation is reduced. The hydrodynamic separation is also effective in combination with filtration separation for waste bleeds, which also reduces the load on filtration separation and can reduce the cost of recovering the liquid medium. As a third embodiment of the cell culture system, FIG. 3 shows a cell culture system configured to utilize the hydrodynamic separation in bleed discharge. In this embodiment, a depth filter is utilized as the filter of the filtration separator to collect the liquid medium from the bleed, and an additional filtration separator is used to remove the cells from the liquid medium containing the cells in culturing and recover the liquid medium.

Specifically, the cell culture system 51 of FIG. 3 has a culture tank 2, a hydrodynamic separation device 3, a filtration separator 4, a medium supplement unit 10, and a depth filter 52. The cell culture system 51 differs from the cell culture system 1 of FIG. 1 in that the hydrodynamic separation device 3 is installed in the discharge path 21b for discharging the bleed and is connected to the depth filter 52. The filtration separator 4 is connected direct to the culture tank 2.

When the cell density of the liquid medium in the culture tank 2 exceeds a predetermined amount, the liquid medium C discharged as the bleed is supplied from the culture tank 2 to the inlet 31 of the hydrodynamic separation device 3 through the discharge path 21b by the driving of the pump 11. Discharge of the bleed can be set based on predicted cell growth, and it can be appropriately modified by monitoring the cell density in the culture tank 2. The supply of the liquid medium to the hydrodynamic separation device is controlled by the pressure-regulating valve 13 and the flow rate-adjusting valve 15 as in the cell culture system of FIG. 1 and FIG. 2, and the pressure gauge 12 and the flow meter 14 can be used to adjust the flow rate (rated flow rate) and the pressure to an appropriate level. In order to supply the bleed at an appropriate flow rate, continuous supply or intermittent supply is performed depending on the situation. In the hydrodynamic separation device 3, the liquid medium C separates into a cell-rich fraction and a cell-poor fraction. The outlet 33 of the hydrodynamic separation device 3 is connected to the depth filter 52 through the supply path 53, and the cell-poor fraction is supplied to the depth filter 52. In this embodiment, in order to temporarily store the cell-poor fraction C', a container 54 capable of preventing microbial contamination is provided in the supply path 53, and it is thus configured that the supply to the depth filter 52 can be adjusted. However, the container 54 may be omitted.

Since the depth filter 52 filters the cell-poor fraction C', which has a low cell density, clogging is unlikely to occur and it is easy to handle the processing capacity of the filter. Therefore, the cells can be removed from the cell-poor fraction C' of the liquid medium supplied from the culture tank 2, and the liquid medium can be efficiently recovered. The hydrodynamic separation device 3 has a discharge path 55 connected to the outlet 32, and the cell-rich fraction is discharged as a bleed from the discharge path 55 to the outside. Since the cells are concentrated on the cell-rich fraction side, the amount of liquid medium discarded as bleed decreases.

On the other hand, the inlet 41 and the outlet 43 of the filtration separator 4 are connected direct to the culture tank 2 by the supply path 56 and the return path 57. The liquid medium C in the culture tank 2 is continuously supplied to the filtration separator 4 by the driving of the pump 58, and the liquid medium C1 that has passed through the filter is collected from the outlet 42. The liquid medium having increased cell density returns from the outlet 43 to the culture tank 2 through the return path 57. As the liquid medium C circulates between the culture tank 2 and the filtration separator 4, the cell density of the liquid medium and the concentration of useful substances increase from the values at the start of the culture. At the initial stage of culturing, the concentration of useful substances in the liquid medium C is low and nutrients such as glucose are not depleted. Therefore, the liquid medium C1 recovered from the filtration separator 4 may be supplied to the culture tank 2 and reused. Thereby the consumption of the liquid medium can be suppressed.

In the cell culture system 51, the liquid medium C in the culture tank 2 is supplied direct to the filtration separator 4. Therefore, in order to supply the liquid medium C having a cell density suitable for filtration separation, the appropriate amount for the cell density of the liquid medium C in the culture tank 2 is the cell density suitable for filtration separation. With this as the predetermined amount, the liquid medium in the culture tank 2 is monitored. Therefore, a configuration in which the liquid medium discharged as bleed from the culture tank 2 is not discarded as it is but is collected through the hydrodynamic separation device and the depth filter 52 is useful in suppressing wasteful disposal of the liquid medium. The depth filter is a filter that captures a filter target not only on the surface but also on the inside, but it can be regarded, structurally, as a combination of a plurality of types of filters or separation membranes having different pore diameters.

In the cell culture system 51 of FIG. 3, the liquid medium C1 is continuously collected from the outlet 42 of the filtration separator 4, and the liquid medium C2 is intermittently collected from the depth filter 52 as a treatment for bleed discharge. A new liquid medium C0 is replenished from the medium tank 16 of the medium supplement unit 10 through the medium replenishment path 17 in accordance with the recovery amount of the liquid medium C1 and the bleed discharge amount, and the amount of the liquid medium in the culture tank 2 is maintained. The surplus cells are obtained as a cell-rich fraction by the hydrodynamic separation and as a filtration residue of the depth filter 52. Since the bleed discharged in the state where the cell growth of the culture tank 2 is active contains abundant useful substances, the recovery of the liquid medium C2 by the depth filter 52 is useful for increasing the recovery rate of useful substances. In this regard, in the cell culture system 1 of FIG. 1, since the cell density of the liquid medium in the culture tank 2 can be set high, recovering the liquid medium from the bleed in the cell culture system 1 is advantageous for improving the recovery efficiency of useful substances. Therefore, if the configuration of FIG. 3 to recover the liquid medium from the bleed using the hydrodynamic separation and the filtration is incorporated into the cell culture system 1 of FIG. 1, it is very effective in reducing the amount of liquid medium discarded and improving the efficiency of recovering useful substances.

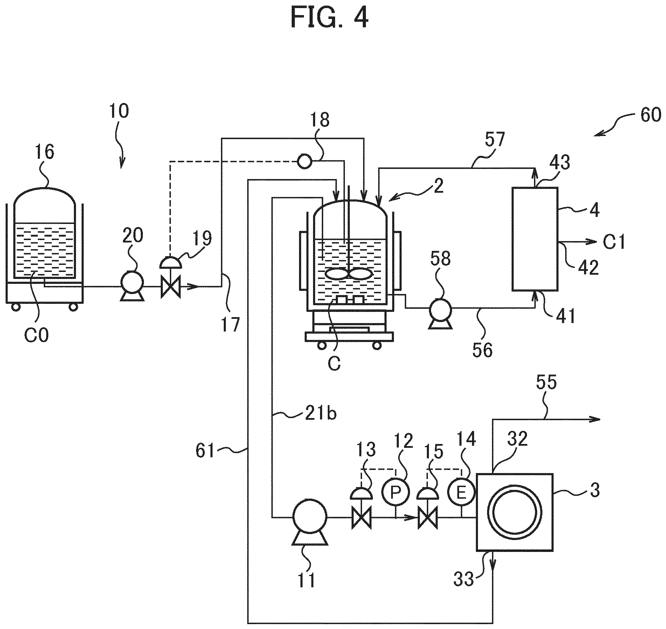

FIG. 4 shows a fourth embodiment of the cell culture system. Also in this embodiment, hydrodynamic separation is utilized in the bleed discharge. However, in the cell culture system 60 of FIG. 4, a medium return path is provided to return the cell-poor fraction obtained by the hydrodynamic separation to the culture tank 2. That is, filtration separation of cell-poor fraction is not performed, but the cell-poor fraction is used to reduce the cell density of the liquid medium in the culture tank.

Specifically, the cell culture system 60 of FIG. 4 has a culture tank 2, a hydrodynamic separation device 3, a filtration separator 4, and a medium supplement unit 10. The culture tank 2 contains a liquid medium containing the cells to be cultured, and the hydrodynamic separation device 3 proceeds the separation process of the liquid medium in the same manner as described above, by utilizing the vortex flow generated by flow through a curved flow channel having a rectangular cross-section. That is, the liquid medium supplied as the bleed from the culture tank 2 is separated into a cell-rich fraction having a relatively high cell density and a cell-poor fraction having a relatively low cell density. Supply of the liquid medium to the hydrodynamic separation device 3 is controlled by the pressure-regulating valve 13 and the flow rate-adjusting valve 15 as in the cell culture systems of FIG. 1 to FIG. 3. Then it can be adjusted to an appropriate flow rate (rated flow rate) by using the pressure gauge 12 and the flow meter 14.

Similar to the cell culture system 51, the filtration separator 4 is connected direct to the culture tank 2 by the supply path 56 and the return path 57, and removes cells from the liquid medium continuously supplied from the culture tank 2, to collect the liquid medium C1 that has passed through the filter. The remaining liquid medium that contains the cells removed by the filter is returned to the culture tank 2. Therefore, as in the case of FIG. 3, the cell density of the liquid medium in the culture tank 2 is adjusted to a range suitable for filtration separation.

The hydrodynamic separation device 3 is installed in the discharge path 21b for discharging the bleed, and the liquid medium discharged as the bleed is supplied to the hydrodynamic separation device 3 by the drive of the pump 11 according to the growth state of the cells in the culture tank 2. Supply of the liquid medium to the hydrodynamic separation device is controlled by the pressure-regulating valve 13 and the flow rate-adjusting valve 15 as in the cell culture systems of FIG. 1 to FIG. 3. Thus it can be adjusted to have appropriate flow rate (rated flow rate) and pressure with use of a pressure gauge 12 and a flow meter 14. The cell-rich fraction of the liquid medium separated in the hydrodynamic separation device 3 is discarded from the discharge path 55 connected to the outlet 32. On the other hand, the outlet 33 of the hydrodynamic separation device is connected to the culture tank 2 by the return path 61, and the cell-poor fraction is supplied to the culture tank 2 through the return path 61. As a result, the cell density of the liquid medium in the culture tank 2 decreases. In this configuration, the weight reduction of the liquid medium associated with bleed drainage corresponds to the amount of cell-rich fraction discarded from the discharge path 55. Therefore, the amount of new liquid medium C0 replenished to maintain the amount of liquid medium in the culture tank 2 decreases as compared with the cell culture system 51 in FIG. 3, and the decrease corresponds to the amount of the cell-poor fraction. Therefore, it is configured to be capable of suppressing an unnecessarily increased amount of the liquid medium consumed for cell culture. Further, by supplying the cell-poor fraction to the culture tank 2 instead of the new liquid medium, it is possible to maintain the cell density of the liquid medium at a predetermined amount while suppressing a decrease in the concentration of useful substances in the culture tank 2. Therefore, the liquid medium recovered from the filtration separator 4 is advantageous in terms of efficiency of separating and purifying useful substances.

The configuration of the above-described embodiments may be modified to use a plurality of hydrodynamic separation devices. By arranging a plurality of hydrodynamic separation devices in series, the liquid medium can be separated in multiple stages. For example, when two hydrodynamic separation devices are arranged in series and the cell-rich fraction discharged from the first-stage hydrodynamic separation device is supplied to the second-stage hydrodynamic separation device, it is possible to obtain a cell-rich fraction in which cells are concentrated at a higher density. This is highly useful in application to bleed discharge and can discard more densely concentrated cells. Further, when the cell-poor fraction is supplied to the second-stage hydrodynamic separation device, a cell-poor fraction having a further reduced cell density can be obtained, so that the efficiency of filtration separation can be improved. Since the liquid medium is separated into three fractions by the two-step hydrodynamic separation, it is possible to carry out separation specialized for disposal, reuse, or recovery of liquid medium for the purpose of each fraction, by adjusting the division ratio in each stage appropriately. Further, by arranging a plurality of hydrodynamic separation devices in parallel, the processing flow rate of the liquid medium can be increased, so that the processing at a flow rate exceeding the rated flow rate is possible.

The cells grow large when the activity is high. However, when the activity decreases, they die and decompose in a relatively small state. Most of the relatively small cells are dead cells or dead cell fragments (debris), and the proportion of active cells in the process of DNA synthesis is small. Hydrodynamic separation is capable of separating relatively large particles from small particles depending on the conditions under which the liquid medium flows. Therefore, a single treatment can separate highly active cells from dead cells or dead cell fragments. However, if the above-mentioned multi-step hydrodynamic separation is used, the separation becomes easy. Then it is also possible to separate a fraction enriched with dead cells, dead cell fragments, and condensates of metabolites, which is discarded as bleed. The separation state can be adjusted by setting of the flow velocity (flow rate) of the supplied liquid medium and the dimensions of the cross-section in the curved flow channel.

In the system design of the cell culture system, since the proper processing flow rate (rated flow rate) in the hydrodynamic separation device is the basis, the cell culture system may be configured so that the capacity of the culture tank 2 and the processing flow rate of the hydrodynamic separation device are properly balanced. In the case where the liquid medium capacity of the culture tank 2 is a relatively small amount such that the medium exchange rate exceeds the appropriate value when the processing in the hydrodynamic separation device is continuously performed, the processing may be performed intermittently. Specifically, the processing amount in the hydrodynamic separation device may be set so that the medium exchange rate becomes an appropriate value. Then, the processing time may be calculated based on the rated flow rate, and the separation process may be performed intermittently at regular intervals in a plurality of times.

Using the cell culture system as described above, a cell culture method can be carried out on various cells, and various useful substances such as proteins and enzymes produced by cultured cells can be recovered and used for manufacturing pharmaceutical products. Specifically, it can be applied to the culture of eukaryotic cells such as animal cells (cells of mammals, birds or insects) and fungal cells (cells of fungi such as Escherichia coli or yeast). For example, Chinese hamster ovary cells, baby hamster kidney (BHK) cells, PER.C.6 cells, myeloma cells, HER cells, etc. can be mentioned. Useful substances obtained by such cell culture include, for example, immunoglobulins (monoclonal antibody or antibody fragment), fusion proteins, insulins, growth hormones, cytokines, interferons, glucagon, albumin, lysosome enzyme, human serum albumin, HPV vaccines, blood coagulation factors, erythropoietins, antibodies such as NS0 and SP2/0. The cell culture may be carried out according to a conventional method based on the culture conditions known for each cell. The liquid medium used for cell culture may be any of synthetic medium, semi-synthetic medium, and natural medium, and a medium suitable for the cells to be cultured may be appropriately selected and used. In general, selective enrichment media or selective isolation media formulated to grow a particular bacteria species are preferably used. It may be appropriately selected and used from commercially available liquid mediums, or it may be prepared using nutrients and purified water according to a known formulation. A differential agent (pH indicator, enzyme substrate, sugar, etc.) for the purpose of inspection, a selective agent for suppressing the growth of unintended microorganisms, and the like may be added as necessary. In order to promote the hydrodynamic separation efficiently, it is preferred to use a liquid medium having a low viscosity.

The useful substance produced by the cultured cells and contained in the liquid medium can be recovered by purifying the liquid medium of the fraction on the inner circumferential side recovered from the hydrodynamic separation device. If the useful substance to be produced is also in the cultured cells, the useful substance can be recovered from the cells without discarding the bleed. The cell culture systems of FIG. 2 to FIG. 4 can recover useful substances from the concentrated cells of the cell-rich fraction discharged from the hydrodynamic separation device, and have an efficient configuration for recovery from the cells.

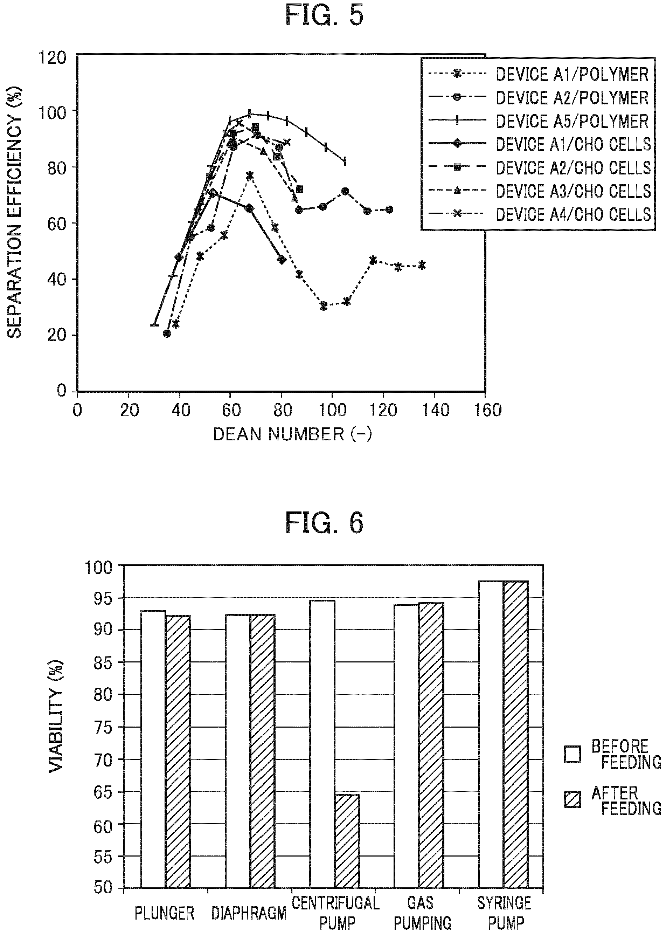

FIG. 5 is a graph showing the results of investigating the relationship between the Dean number and the separation efficiency in the hydrodynamic separation device. In FIG. 5, the hydrodynamic separation has been performed by using any of the five types of hydrodynamic separation devices (devices A1 to A5) having different flow channel dimensions. The results of conducting the separation are shown with use of a separation target of either polymer particles (styrene-divinylbenzene copolymer) or CHO cells (Chinese hamster ovary cells). The average particle size of each separation target is in the range of 14 to 18 µm. The separation efficiency is a value calculated as [1 - (x/X)] × 100 (%). In the calculation formula, X indicates the concentration of the separation target contained in the liquid before separation, and x is the concentration of the separation target contained in the fraction on the inner circumferential side after separation. Since the particle size distribution of each of the separation targets is narrow, the separation efficiency is based on such evaluation that the separation efficiency in a state where the entire amount of particles or cells is concentrated in the outer circumferential fraction is regarded as 100 %. As can be seen from the graph, in both polymer particles and animal cells, the separation efficiency is highest when the Dean number is around 70, and generally, high separation efficiency can be achieved under conditions where the Dean number is in the range of 50 to 80. Therefore, it can be understood that the cell-poor fraction on the inner circumferential side obtained from the hydrodynamic separation device is suitable for filtration separation.

FIG. 6 shows the results of examining the effect on cell viability depending on the type of pump that sends the liquid medium to the hydrodynamic separation device. The liquid medium in which the cells were cultured was supplied to the hydrodynamic separation device using a pump at a discharge pressure of 0.3 MPa, and two fractions of the liquid medium discharged from the separation device were collected together. A small amount of the collected liquid medium was sampled, and the viability (%, the ratio of living cells in all cells) of the sample cells was measured with a cell measuring device (manufactured by Beckman Coulter, product name: Vi-Cell). The result of measurement is shown in the graph of FIG. 6. "Gas pumping" in the graph is a form in which the liquid medium is pressure-fed with compressed air from a pressurizing tank connected with a compressor, and it can be classified as a positive displacement pump. It can be seen from FIG. 6 that the centrifugal pump has a large damage to cells, and that the other pumps classified as positive displacement pumps prevent the decrease in the viability.

The hydrodynamic separation can promote concentrated separation of cells much more efficiently than centrifugation, and can concentrate and separate the cells with a high viability without damaging the cells as compared with the filtration separation and the like. As described above, the hydrodynamic separation technique can be used to separate the liquid medium under the culturing into a cell-rich fraction with a relatively high cell density and a cell-poor fraction with a relatively low cell density. Therefore, with use of the cell-poor fraction, it becomes possible to recover the liquid medium efficiently by filtration separation. By purifying the recovered liquid medium, useful components can be collected. Further, in the treatment of bleed, a depth filter can be applied by using the cell-poor fraction, and a liquid medium with a high concentration of useful substances can be recovered. Therefore, the production efficiency of useful substances can be improved. In addition, by discarding the cell-rich fraction in which cells are concentrated, the amount of liquid medium that is wasted unnecessarily can be reduced. In the cell culture system of the present disclosure, since the liquid medium in the hydrodynamic separation is supplied to the curved flow channel at a relatively high speed, the processing capacity for concentrated separation of cells is high and it is sufficiently applicable to practical-scale cell culture. Since the cells can be recovered with a high viability in the hydrodynamic separation, the cell culture can be continued while maintaining the high proliferative power by supplying a new liquid medium together with the recovered cells to the culture tank.

### Industrial Applicability

It is possible to eliminate clogging in filtration separation for removing cells from the liquid medium in which the cells are cultured, and to realize efficient recovery of the liquid medium and production of useful substances. By applying to the manufacture of pharmaceuticals using biotechnology, it contributes to the improvement of economy and quality in the provision of products such as hormones, cytokines, enzymes, antibodies, vaccines, etc., and it will be possible to promote the spread and generalization of medicines that are rare or expensive at present.

### Explanation of Reference

- 1, 50, 51, 60: cell culture system
- 2: culture tank
- 3: hydrodynamic separation device
- 5: filtration separator
- 5: liquid feeding unit
- 6, 8, 53, 56: supply path
- 7: first return path
- 9: second return path
- 10: medium supplement unit
- 11, 58: pump
- 12: pressure gage
- 13: pressure-regulating valve
- 14: flow meter
- 15, 19: flow rate-adjusting valve
- 16: medium tank
- 17: medium replenishment path
- 18: liquid level meter
- 20: tubing pump
- 21, 21a, 55: discharge path
- 22, 22a: on-off valve
- 31, 41: inlet
- 32, 33, 42, 43: outlet
- 52: depth filter
- 54: container
- 51, 61: return path
- C, C0, C1, C2: liquid medium
- C': cell-poor fraction

## Claims

1. A cell culture system, comprising:
a culture tank that contains a liquid medium containing cells to be cultured;
a hydrodynamic separation device having a curved flow channel having a rectangular cross-section, and separating the liquid medium supplied from the culture tank into a cell-rich fraction having a relatively high cell density and a cell-poor fraction having a relatively low cell density, using a vortex flow generated by flow through the curved flow channel; and
a filtration separator having a filter to remove cells from the cell-poor fraction separated by the hydrodynamic separation device and recover the liquid medium.

2. The cell culture system according to claim 1, further comprising:
a first return path connecting the hydrodynamic separation device and the culture tank to supply the cell-rich fraction to the culture tank; and
a second return path connecting the hydrodynamic separation device and the culture tank to supply the cells removed from the cell-poor fraction to the culture tank.

3. The cell culture system according to claim 2, further comprising:
a discharge path to discharge a part of the liquid medium containing the cells to the outside as a bleed.

4. The cell culture system according to claim 3, wherein the discharge path is provided as a branch from the first return path, to discard a part or the entire of the cell-rich fraction.

5. The cell culture system according to claim 2, wherein the filter of the filtration separator is a depth filter, and further comprising:
a discharge path to discard the cell-rich fraction to the outside as a bleed.

6. The cell culture system according to claim 5, further comprising:
a medium replenishment path for supplying a new liquid medium to the culture tank; and
an additional filtration separator having a filter for removing the cells from the liquid medium supplied from the culture tank and collecting the liquid medium.

7. A cell culture system, comprising:
a culture tank that contains a liquid medium containing cells to be cultured;
a hydrodynamic separation device having a curved flow channel having a rectangular cross-section, and separating the liquid medium supplied from the culture tank into a cell-rich fraction having a relatively high cell density and a cell-poor fraction having a relatively low cell density, using a vortex flow generated by flow through the curved flow channel;
a medium return path that connects the hydrodynamic separation device and the culture tank and supplies the cell-poor fraction to the culture tank to reduce the cell density of the liquid medium in the culture tank; and
a filtration separator having a filter to remove the cells from the liquid medium supplied from the culture tank and recover the liquid medium.

8. The cell culture system according to claim 7, further comprising:
a return path that connects the filtration separator and the culture tank and supplies a residual liquid medium containing the cells removed by the filtration separator, to the culture tank.

9. The cell culture system according to any one of claims 1 to 8, wherein the hydrodynamic separation device has a single inlet for taking in the liquid medium and at least two outlets for discharging the liquid medium separated, wherein the cell-rich fraction is discharged from one of the outlets, and the cell-poor fraction is discharged from the other outlet.

10. The cell culture system according to any one of claim 1 to 9, further comprising:
an urging device that urges the liquid medium with a flow pressure for supplying the liquid medium to the hydrodynamic separation device; and
a pressure control mechanism that controls pressure environment so that the pressure difference between the liquid medium introduced to the hydrodynamic separation device and the liquid medium derived from the hydrodynamic separation device is equal to or less than a predetermined value.

11. A cell culture method, comprising:
a cell culture for culturing cells in a liquid medium;
a hydrodynamic separation to separate the liquid medium supplied from the cell culture into a cell-rich fraction having a relatively high cell density and a cell-poor fraction having a relatively low cell density, using a vortex flow generated by flow through a curved flow channel having a rectangular cross-section; and
a filtration separation in which the cells are removed from the cell-poor fraction separated by the hydrodynamic separation with a filter, and the liquid medium is recovered.

12. A cell culture method, comprising:
a cell culture for culturing cells in a liquid medium;
a hydrodynamic separation to separate the liquid medium supplied from the cell culture into a cell-rich fraction having a relatively high cell density and a cell-poor fraction having a relatively low cell density, using a vortex flow generated by flow through a curved flow channel having a rectangular cross-section;
a medium return in which the cell-poor faction is supplied to the cell culture to reduce the cell density of the liquid medium in the cell culture; and
a filtration separator in which cells are removed from the liquid medium supplied from the cell culture with a filter and the liquid medium is recovered.
